# EUROPEAN PATENT SPECIFICATION

(11) **EP 4 398 259 B1**
(45) Date of publication and mention of the grant of the patent: **03.09.2025**
(21) Application number: 24150080.0
(22) Date of filing: 02.01.2024
(51) Int. Cl.: G16H 30/40, G16H 50/20

(54) **SERVER AND METHOD FOR PREDICTING INCIDENCE OF MOYAMOYA DISEASE THROUGH LEARNING OF FUNDUS IMAGE**
SERVER UND VERFAHREN ZUR VORHERSAGE DES AUFTRETENS VON MOYAMOYA-KRANKHEIT DURCH LERNEN DES FUNDUSBILDES
SERVEUR ET PROCÉDÉ DE PRÉDICTION DE L'INCIDENCE D'UNE MALADIE DE MOYAMOYA PAR APPRENTISSAGE D'UNE IMAGE DE FOND D'OEIL

(30) Priority: 03.01.2023 KR 20230000520; 28.03.2023 KR 20230040570
(43) Date of publication of application: 10.07.2024
(73) Proprietor: Industry-Academic Cooperation Foundation, Yonsei University, Seoul 03722 (KR)
(72) Inventor: PARK, Yu Rang, 05507 Seoul (KR); HONG, Jae Seong, 03010 Seoul (KR); SHIM, Kyu Won, 01192 Seoul (KR); YOON, Sang Chul, 04064 Seoul (KR)
(74) Representative: dompatent von Kreisler Selting Werner - Partnerschaft von Patent- und Rechtsanwälten mbB

(56) References cited:
- AKIYAMA YUKINORI ET AL: "Deep Learning-Based Approach for the Diagnosis of Moyamoya Disease", JOURNAL OF STROKE AND CEREBROVASCULAR DISEASES, DEMOS PUBLICATIONS, NEW YORK, NY, US, vol. 29, no. 12, 25 September 2020 (2020-09-25), XP086350680, ISSN: 1052-3057, [retrieved on 20200925], DOI: 10.1016/J.JSTROKECEREBROVASDIS.2020.105322
- LO CHUNG-MING ET AL: "Assessing Ischemic Stroke with Convolutional Image Features in Carotid Color Doppler", ULTRASOUND IN MEDICINE AND BIOLOGY, NEW YORK, NY, US, vol. 47, no. 8, 15 May 2021 (2021-05-15), pages 2266 - 2276, XP086677566, ISSN: 0301-5629, [retrieved on 20210515], DOI: 10.1016/J.ULTRASMEDBIO.2021.03.038
- JAESEONG HONG: "Screening of Moyamoya Disease From Retinal Photographs: Development and Validation of Deep Learning Algorithms", STROKE, vol. 55, no. 3, 1 March 2024 (2024-03-01), US, pages 715 - 724, XP093160324, ISSN: 0039-2499, DOI: 10.1161/STROKEAHA.123.044026

## Description

### BACKGROUND

Embodiments of the present disclosure described herein relate to an electronic device, and more particularly, relate to a server and method for predicting incidence of a moyamoya disease through learning of a fundus image.

A moyamoya disease refers to a disease in which arteries that supply blood to a brain gradually narrow. In particular, the moyamoya disease refers to a special cerebrovascular disease in which a specific portion is blocked as inner walls of both cerebral blood vessels thicken. Stenosis or occlusion is seen at the beginning of the anterior and middle cerebral arteries, and the moyamoya vessels are observed in their vicinity. The moyamoya disease is a rare disease diagnosed in about 200 people per year. However, the number of cases of a moyamoya disease is increasing every year. In children with moyamoya disease, transient ischemic attacks occur. For example, paralysis of one limb occurs within 1 to 2 hours, motor function is paralyzed, pronunciation problems occur, or deterioration of vision occurs. In adults who develop moyamoya disease, cerebral hemorrhage may occur, and there is a high risk of permanent disability. Moreover, when a person eats hot food, blows a wind instrument or balloon, or cries violently, hyperventilation temporarily lowers the concentration of carbon dioxide in the blood and reduces cerebral blood flow, which may lead to the symptoms described above. When symptoms of cerebral ischemia appear, brain protection treatment may be considered by alleviating symptoms with medical medication. When cerebral ischemic symptoms occur repeatedly, surgical treatment may be preferentially considered, and vascular anastomosis may be performed. The younger a person is, the more severe the clinical symptoms 1 are and the faster the disease progresses. Accordingly, it is necessary to actively administer treatment when symptoms are mild.

The moyamoya disease may only be diagnosed through specialized tests such as magnetic resonance imaging (MRI) or computed tomography (CT). In this context, the paper "Deep Learning-Based Approach for the Diagnosis of Moyamoya Disease" by AKIYAMA YUKINORI ET AL, JOURNAL OF STROKE AND CEREBROVASCULAR DISEASES, DEMOS PUBLICATIONS, NEW YORK, NY, US, vol. 29, no. 12, 25 September 2020, discloses such a specialized MRI test.

The moyamoya disease is completely dependent on surgery, and thus early detection and early treatment are important. However, detailed examinations such as MRI or CT may take a lot of time and cost money.

In the meantime, when the moyamoya disease occurs, arterial stenosis occurs not only in the intracranial arteries but also systemically and may also occur in the fundus. The retina may correspond to an extension of the central nervous system and may have similarities with the brain and spinal cord in terms of anatomy. There is a need for a method of quickly and accurately predicting the moyamoya disease through retinal photography, which is a technique of capturing the retina.

### SUMMARY

Embodiments of the present disclosure provide a method of quickly and accurately predict the incidence of a moyamoya disease by using machine learning that learns retinal images.

Problems to be solved by the present disclosure are not limited to the problems mentioned above, and other problems not mentioned will be clearly understood by those skilled in the art from the following description.

According to an embodiment, a server includes a memory that stores a deep learning-based incidence prediction model, and a processor that predicts an incidence probability of a moyamoya disease of a patient from a fundus image by using the incidence prediction model. The processor labels input fundus images as a moyamoya disease patient with the moyamoya disease and a normal patient without the moyamoya disease, learns the incidence prediction model by recognizing fundus images labeled as the moyamoya disease patient and fundus images labeled as the normal patient into the incidence prediction model, and predicts the incidence probability of the moyamoya disease by inputting the new fundus image into the learned incidence prediction model when a new fundus image is input.

According to an embodiment, a method of predicting an incidence probability of a moyamoya disease in a patient from a fundus image by using a deep learning-based incidence prediction model includes labeling input fundus images as a moyamoya disease patient with the moyamoya disease and a normal patient without the moyamoya disease, learning the incidence prediction model by recognizing fundus images labeled as the moyamoya disease patient and fundus images labeled as the normal patient into the incidence prediction model, and predicting the incidence probability of the moyamoya disease by inputting a new fundus image thus input into the learned incidence prediction model.

Besides, a computer program stored in a computer-readable recording medium for execution to implement the present disclosure may be further provided.

### BRIEF DESCRIPTION OF THE FIGURES

The above and other objects and features will become apparent from the following description with reference to the following figures, wherein like reference numerals refer to like parts throughout the various figures unless otherwise specified, and wherein:
FIG. 1 is a diagram for describing a system, according to an embodiment of the present disclosure;
FIG. 2 is a diagram for describing a server, according to an embodiment of the present disclosure;
FIG. 3 is a flowchart for describing a method, according to embodiments of the present disclosure;
FIG. 4 is a diagram for describing an embodiment of preprocessing a fundus image by removing metadata and text data;
FIG. 5 is a diagram for describing an embodiment of preprocessing a fundus image by enhancing the contrast of an image;
FIG. 6 is a diagram for describing a process of preprocessing a fundus image;
FIG. 7 is a graph showing a distribution of fundus images of patients with moyamoya diseases and normal patients according to age;
FIG. 8 is a diagram for describing an embodiment of setting a train dataset for a fundus image;
FIG. 9 is a diagram for describing an embodiment of setting a fold dataset for a fundus image;
FIG. 10 is a diagram for describing a process of modeling an incidence prediction model;
FIG. 11 is a diagram illustrating a score according to a data type indicating a ratio of 1:1;
FIG. 12 is a diagram illustrating a score according to a data type indicating a ratio of 1:2;
FIG. 13 is a diagram illustrating a score according to a data type indicating a ratio of 1:3;
FIG. 14 is a diagram illustrating a ROC curve for each data type;
FIG. 15 is a diagram illustrating a ROC curve for each data type according to a plurality of fold datasets;
FIG. 16 is a diagram showing fundus images for each stage;
FIG. 17 is a diagram for describing a plurality of stages;
FIG. 18 is a diagram for describing a process of modeling an incidence prediction model according to a data type indicating a ratio of 1:3;
FIG. 19 is a diagram showing a best score for each target stage range;
FIG. 20 is a diagram showing an average score for each target stage range;
FIG. 21 is a diagram showing a best score for each target stage; and
FIG. 22 is a diagram showing an average score for each target stage.

### DETAILED DESCRIPTION

The same reference numerals denote the same elements throughout the present disclosure. The present disclosure does not describe all elements of embodiments. Well-known content or redundant content in which embodiments are the same as one another will be omitted in a technical field to which the present disclosure belongs. A term such as 'unit, module, member, or block' used in the specification may be implemented with software or hardware. According to embodiments, a plurality of 'units, modules, members, or blocks' may be implemented with one component, or a single 'unit, module, member, or block' may include a plurality of components.

Throughout this specification, when it is supposed that a portion is "connected" to another portion, this includes not only a direct connection, but also an indirect connection. The indirect connection includes being connected through a wireless communication network.

Furthermore, when a portion "comprises" a component, it will be understood that it may further include another component, without excluding other components unless specifically stated otherwise.

Throughout this specification, when it is supposed that a member is located on another member "on", this includes not only the case where one member is in contact with another member but also the case where another member is present between two other members.

Terms such as 'first', 'second', and the like are used to distinguish one component from another component, and thus the component is not limited by the terms described above.

Unless there are obvious exceptions in the context, a singular form includes a plural form.

In each step, an identification code is used for convenience of description. The identification code does not describe the order of each step. Unless the context clearly states a specific order, each step may be performed differently from the specified order.

Hereinafter, operating principles and embodiments of the present disclosure will be described with reference to the accompanying drawings.

In this specification, a 'system according to an embodiment of the present disclosure' includes all various devices capable of providing results to a user by performing arithmetic processing. For example, the system according to an embodiment of the present disclosure may include all of a computer, a server device, and a portable terminal, or may be in any one form.

Here, for example, the computer may include a notebook computer, a desktop computer, a laptop computer, a tablet PC, a slate PC, and the like, which are equipped with a web browser.

A server device may be a server that processes information by communicating with an external device and may include an application server, a computing server, a database server, a file server, a game server, a mail server, a proxy server, and a web server.

For example, the portable terminal may be a wireless communication device that guarantees portability and mobility, and may include all kinds of handheld-based wireless communication devices such as a smartphone, a personal communication system (PCS), a global system for mobile communication (GSM), a personal digital cellular (PDC), a personal handyphone system (PHS), a personal digital assistant (PDA), International Mobile Telecommunication (IMT)-2000, a code division multiple access (CDMA)-2000, W-Code Division Multiple Access (W-CDMA), and Wireless Broadband Internet terminal (Wibro) terminal, and a wearable device such as a timepiece, a ring, a bracelet, an anklet, a necklace, glasses, a contact lens, or a head-mounted device (HMD).

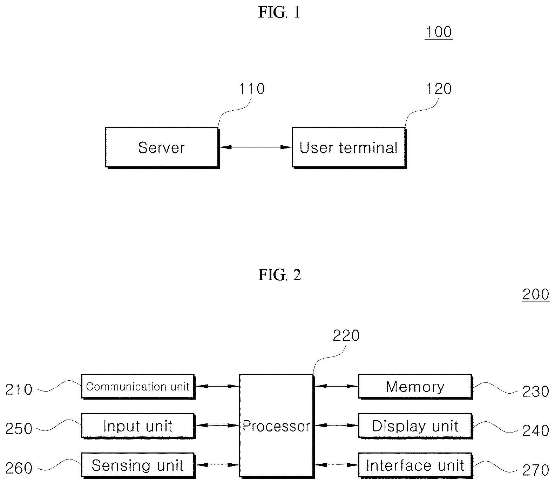

FIG. 1 is a diagram for describing a system, according to an embodiment of the present disclosure.

Referring to FIG. 1, a system 100 may include a server 110 and a user terminal 120.

The server 110 may include an application server, a computing server, a database server, a file server, a game server, a mail server, a proxy server, and a web server. The server 110 may provide the user terminal 120 with an application that predicts the incidence probability of a moyamoya disease in a patient from a fundus image by using a deep learning-based incidence prediction model through a communication network. The server 110 may transmit, to the user terminal 120, information for predicting the incidence probability of a moyamoya disease in a patient from a fundus image by using a deep learning-based incidence prediction model.

The user terminal 120 may download the application from the server 110 over a communication network. In some embodiments, the communication network may be a secure network capable of being accessed by only authorized users. The user terminal 120 may execute the downloaded application. The user terminal 120 may upload a fundus image to the server 110. The server 110 may predict the incidence probability of a patient's moyamoya disease by entering the fundus image into a deep learning-based onset prediction model. The user terminal 120 may obtain information for predicting the incidence probability of the patient's moyamoya disease from the server 110.

As described above, the incidence of a moyamoya disease may be quickly and accurately predicted by using machine learning that learns retinal images.

FIG. 2 is a diagram for describing a server, according to an embodiment of the present disclosure.

Referring to FIG. 2, a server 200 may include a communication unit 210, a processor 220, a memory 230, a display unit 240, an input unit 250, a sensing unit 260, and an interface unit 270.

The communication unit 210 may communicate with the outside. For example, the communication unit 210 may communicate with the user terminal 120 of FIG. 1. The communication unit 210 may include one or more components capable of communicating with an external device, and may include, for example, at least one of a broadcast reception module, a wired communication module, a wireless communication module, a short-range communication module, and a location information module.

The communication unit 210 may receive a fundus image from the user terminal 120.

The processor 220 may perform an algorithm for controlling operations of components within the server 200. The processor 220 may control the memory 230, which stores data for a program that implements the algorithm. The processor 220 may perform the above-described operations by using data stored in the memory 230. In this case, each of the memory 230 and the processor 220 may be implemented as separate chips. Alternatively, the memory 230 and the processor 220 may be implemented as a single chip. Furthermore, the processor 220 may control one of the components described above or the combination of the components to implement various embodiments of the present disclosure described below in the drawings on the server 200.

The processor 220 may predict the incidence probability of a patient's moyamoya disease from the fundus image by using an incidence prediction model stored in the memory 230.

The processor 220 may label the input fundus images as patients with moyamoya diseases and normal patients without moyamoya diseases. Moreover, the processor 220 may learn an incidence prediction model by recognizing fundus images labeled as moyamoya disease patients and fundus images labeled as normal patients into the incidence prediction model. When a new fundus image is input, the processor 220 may predict the incidence probability of the moyamoya disease by entering the new fundus image into the learned incidence prediction model.

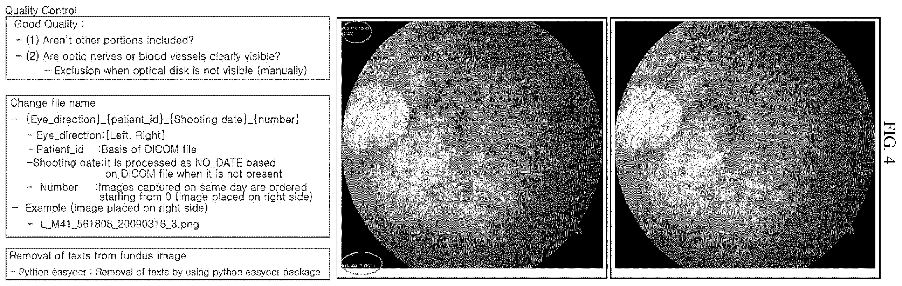

In some embodiments, the processor 220 may preprocess the input fundus images. For example, a method of preprocessing a fundus image may include removing metadata and text data included in the fundus image, using only a fundus image, which includes an optic disk, from among fundus images for learning, performing gray scaling on the fundus image, and/or enhancing the contrast of the fundus image. Descriptions of these embodiments are given below with reference to FIGS. 4 to 6.

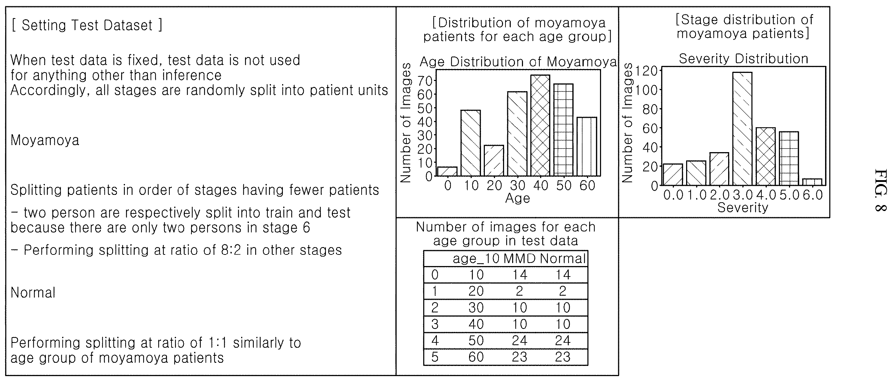

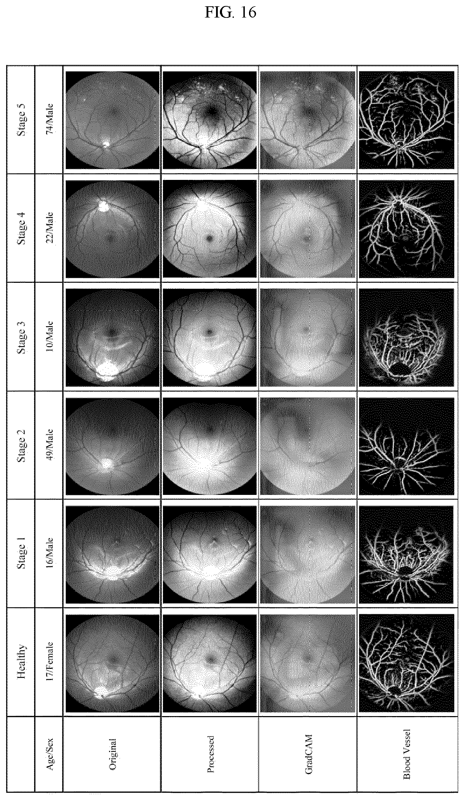

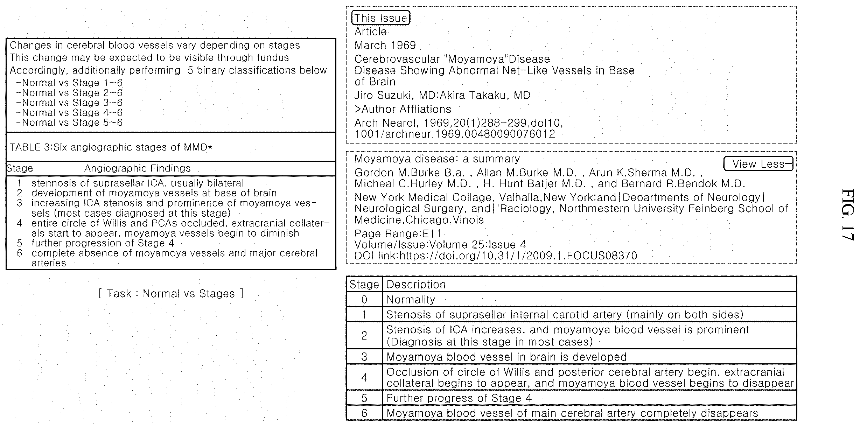

In some embodiments, the processor 220 may split fundus images labeled in the order of stages, which have fewer patients for each age group, from among a plurality of stages classified depending on severity into a train dataset. In this case, the processor 220 may split 80% of fundus images labeled as patients with moyamoya diseases into a training dataset of the train dataset, and may split 20% of fundus images into a test dataset of the train dataset. Moreover, the processor 220 may split fundus images labeled as normal patients into the train dataset at a ratio of 1:1 between the number of fundus images labeled as normal patients and the number of fundus images labeled as patients with moyamoya diseases, which are split for each age group. Descriptions of these embodiments are given below with reference to FIG. 8. Descriptions of stages will be given later with reference to FIGS. 16 and 17.

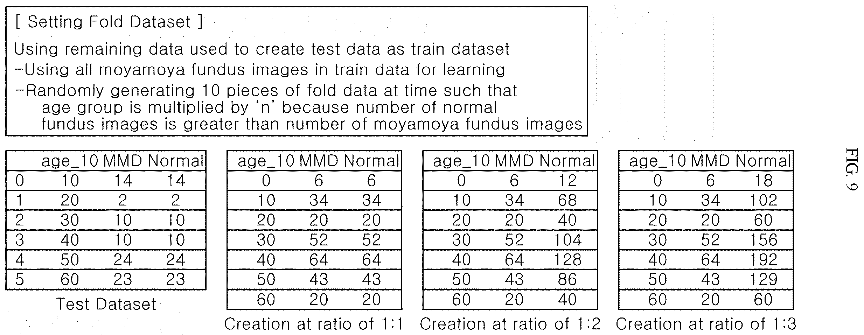

In some embodiments, the processor 220 may split the fundus images labeled as normal patients into a fold dataset at a ratio of 1:n ('n' is an integer greater than or equal to 1) between the fundus images labeled as normal patients and the number of fundus images labeled as patients with moyamoya diseases, which are split for each age group. In this case, the processor 220 may generate a plurality of fold datasets. For example, the number of fold datasets may be 10, but is not limited thereto. Descriptions of these embodiments are given below with reference to FIG. 9.

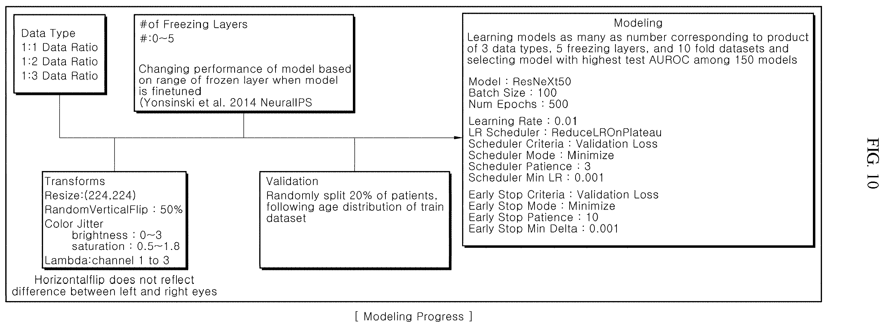

In some embodiments, the processor 220 may learn a plurality of incidence prediction models as many as 'n' data types, each of which indicates the ratio of 1:n, the number of freezing layers for freezing layers when the incidence prediction model is finetuned, and the number of the fold dataset. Besides, the processor 220 may select an incidence prediction model, which has the highest area under receiver operating characteristic (AUROC), from among the plural incidence prediction models. Descriptions of these embodiments are given below with reference to FIGS. 10 to 15.

In some embodiments, the fundus image may include nerves, ocular substances, and blood vessels. The processor 220 may determine the moyamoya disease based on at least one of the thickness of a blood vessel and the number of blood vessels included in the new fundus image of the moyamoya disease patient, and a predetermined reference value. For example, the processor 220 may determine whether the thickness value of a blood vessel in the fundus image is greater than the reference value. When the thickness value of a blood vessel is greater than the reference value, a patient may be determined to be suffering from the moyamoya disease. For another example, the processor 220 may determine whether the number of blood vessels in the fundus image is greater than the reference value (number). When the number of blood vessels is greater than the reference value, a patient may be determined to be suffering from the moyamoya disease. Descriptions of these embodiments are given below with reference to FIG. 16.

In some embodiments, in the case of normal patients, an operation of determining a moyamoya disease may be performed based on the entire fundus image. In detail, for example, the processor 220 may determine a moyamoya disease based on the whole new fundus image of a normal patient. Descriptions of these embodiments are given below with reference to FIG. 16.

The memory 230 may store a deep learning-based incidence prediction model.

The display unit 240 may have a mutual layer structure with the touch sensor or may be integrally formed with the touch sensor. Accordingly, a touch screen may be implemented. The display unit 240 may display information processed in the server 200.

The input unit 250 may be used to enter image information (or signal), audio information (or signal), data, or information entered by a user. The input unit 250 may include at least one of at least one camera, at least one microphone, and a user input unit.

The sensing unit 260 senses at least one of information of the server 200, surrounding environment information surrounding the server 200, and user information, and generates a sensing signal corresponding to the sensed result.

The interface unit 270 serves as an interface for various types of external devices connected to the server 200.

As described above, the incidence of a moyamoya disease may be quickly and accurately predicted by using machine learning that learns retinal images.

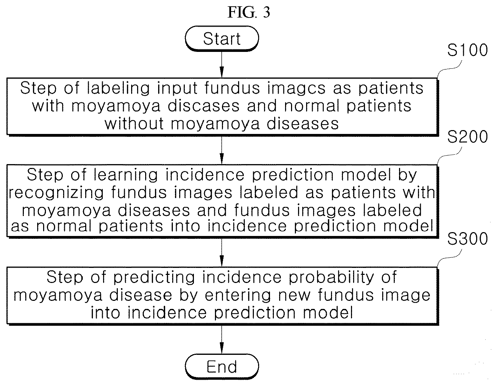

FIG. 3 is a flowchart for describing a method, according to embodiments of the present disclosure.

Referring to FIG. 3, a method of FIG. 3 may be performed by the server 200 of FIG. 2. The method may refer to a method of predicting the incidence probability of a moyamoya disease in a patient from a fundus image by using a deep learning-based incidence prediction model.

A step of labeling input fundus images as patients with moyamoya diseases and normal patients without moyamoya diseases is performed (S100).

A step of learning an incidence prediction model by recognizing fundus images labeled as the patients with moyamoya diseases and fundus images labeled as the normal patients into the incidence prediction model (S200).

In some embodiments for step S200, labeled fundus images may be split into a train dataset. In this case, the split order may be the order of stages, which have the small number of patients for each age group, from among a plurality of stages classified depending on severity.

In some embodiments for step S200, 80% of the fundus images labeled as the patients with moyamoya diseases may be split into a training dataset of the train dataset. Moreover, 20% of the fundus images labeled as patients with moyamoya diseases may be split into the test dataset of the train dataset.

In some embodiments for step S200, the fundus images labeled as the normal patients into the train dataset may be split at a ratio of 1:1 between the number of fundus images labeled as the normal patients and the number of fundus images labeled as patients with moyamoya diseases, which are for each age group.

In some embodiments for step S200, the fundus images labeled as normal patients may be split into a fold dataset at a ratio of 1:n ('n' is an integer greater than or equal to 1) between the fundus images labeled as normal patients and the number of fundus images labeled as patients with moyamoya diseases, which are split for each age group. Moreover, two or more fold datasets may be created. In other words, the fold dataset may be created repeatedly.

In some embodiments for step S200, a plurality of incidence prediction models may be learned as many as 'n' data types, each of which indicates the ratio of 1:n, the number of freezing layers for freezing layers when the incidence prediction model is finetuned, and the number of the fold dataset. Besides, an incidence prediction model having the highest AUROC may be finally selected from among the plural incidence prediction models.

A step of predicting the incidence probability of the moyamoya disease by entering a new fundus image into the incidence prediction model is performed (S300).

In some embodiments for step S300, the moyamoya disease may be determined based on a predetermined reference values and blood vessels included in the new fundus image of the patient's moyamoya disease.

In some embodiments for step S300, the moyamoya disease may be determined based on a predetermined reference thickness, and the thickness of a blood vessel included in the new fundus image of the patient's moyamoya disease.

In some embodiments for step S300, the moyamoya disease may be determined based on a predetermined reference number and the number of blood vessels included in the new fundus image of the patient's moyamoya disease.

In some embodiments for step S300, the moyamoya disease may be determined based on the total of the new fundus image of the normal patient.

In some embodiments, the method of FIG. 3 may further include preprocessing the input fundus images. For example, the step of preprocessing fundus images may be performed before step S100. For example, the step of preprocessing a fundus image may include removing metadata and text data, using only a fundus image including an optic disk for learning, performing gray scaling on the image, and enhancing the contrast of the image. However, an embodiment is not limited thereto.

As described above, the incidence of a moyamoya disease may be quickly and accurately predicted by using machine learning that learns retinal images.

FIG. 4 is a diagram for describing an embodiment of preprocessing a fundus image by removing metadata and text data.

Referring to FIGS. 2 and 4, the processor 220 may determine whether other portions other than a fundus are not included in a fundus image. The processor 220 may determine whether optic nerves or blood vessels are clearly visible in the fundus image. When it is determined that the optic disk is not visible within the fundus image, the processor 220 may exclude the corresponding fundus image.

The processor 220 may change a file name of the fundus image as shown in the example in FIG. 4. The file name may include an eye direction, an identifier of a patient, a shooting date, an image number, or the like.

The processor 220 may remove text data including the fundus image. For example, the processor 220 may remove a text image from the fundus image by using EasyOCR open source that is a Python package.

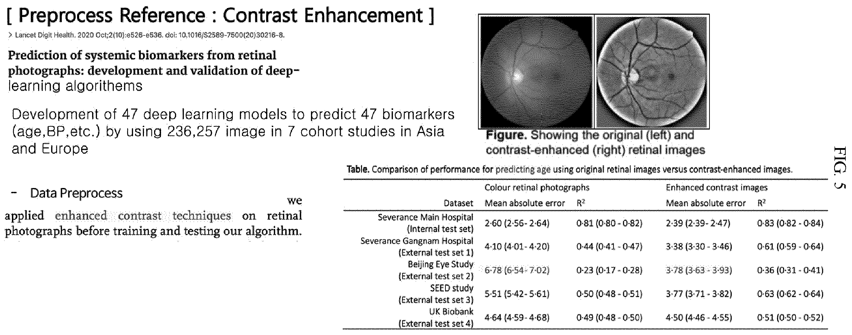

FIG. 5 is a diagram for describing an embodiment of preprocessing a fundus image by enhancing the contrast of an image.

Referring to FIGS. 2 and 5, the processor 220 may predict systemic biomarkers from fundus images generated by retinal photography for the development and verification of a deep-learning algorithm.

To this end, the processor 220 may apply enhanced contrast techniques to a fundus image generated by retinal photography before training an incidence prediction model and testing the trained incidence prediction model.

As Table shown in FIG. 5, when the contrast of the fundus image is enhanced, the cross entropy loss of a value predicted by the incidence prediction model may decrease. Accordingly, the accuracy of the incidence prediction model may be increased.

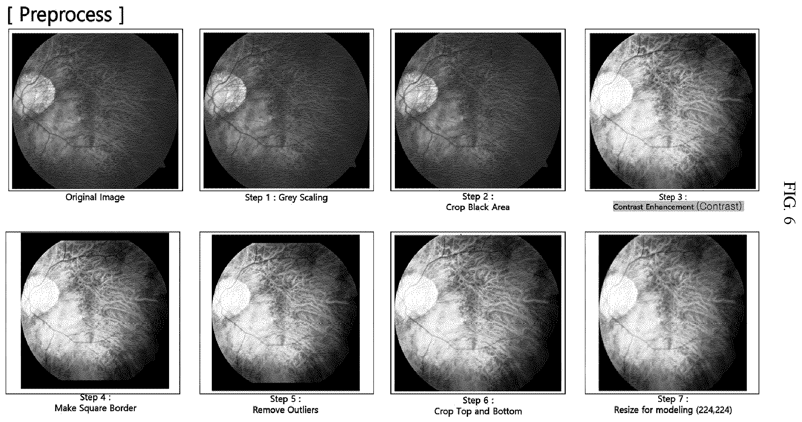

FIG. 6 is a diagram for describing a process of preprocessing a fundus image.

Referring to FIGS. 2 and 6, in a first step (Step 1), the processor 220 may perform image gray scaling on an original fundus image.

In a second step (Step 2), the processor 220 may crop a black area in the grayscaled fundus image.

In a third step (Step 3), the processor 220 may enhance the contrast of the fundus image in which the black area is cropped.

In a fourth step (Step 4), the processor 220 may mask a square border in a fundus image with enhanced contrast.

In a fifth step (Step 5), the processor 220 may remove an outlier from the masked fundus image.

In a sixth step (Step 6), the processor 220 may crop an upper portion and a lower portion of the fundus image from which the outlier is removed.

In a seventh step (Step 7), the processor 220 may resize the fundus image for modeling.

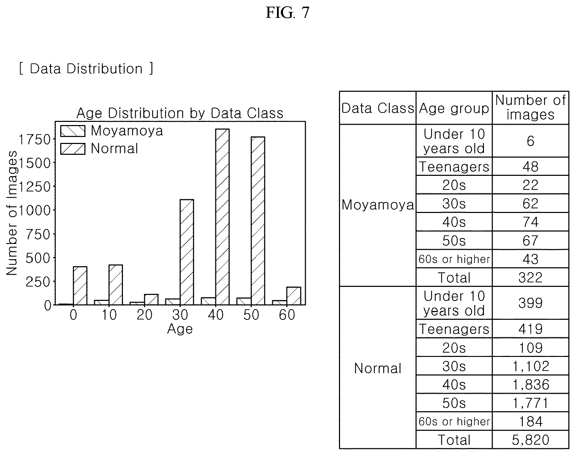

FIG. 7 is a graph showing a distribution of fundus images of patients with moyamoya diseases and normal patients according to age.

Referring to FIG. 7, a patient's age may be classified into under 10 years old, teenagers, 20s, 30s, 40s, 50s, and 60s. Fundus images of patients with moyamoya diseases and fundus images of normal patients may be collected for each age. For example, a total of 322 fundus images (e.g., 6 fundus images, 48 fundus images, 22 fundus images, 62 fundus images, 74 fundus images, 67 fundus images, and 43 fundus images) of the patients with moyamoya diseases may be collected depending on the age of the patients with moyamoya diseases. In the meantime, a total of 5,820 fundus images (e.g., 399 fundus images, 419 fundus images, 109 fundus images, 1,102 fundus images, 1,836 fundus images, 1,771 fundus images, and 184 fundus images) of the normal patients may be collected depending on the age of the normal patients.

FIG. 8 is a diagram for describing an embodiment of setting a train dataset for a fundus image.

Referring to FIGS. 2 and 8, when test data is fixed, the test data may never be used for anything other than inference. Accordingly, an input fundus images may be randomly split into patient units of all stages. In other words,

In the case of patients with moyamoya diseases, a processor may split fundus images labeled in the order of stages, which have fewer patients for each age group into a train dataset. The processor 220 may split fundus images labeled as patients with moyamoya diseases at a ratio of 8:2 between a training dataset of the train dataset and a test dataset of the train dataset. When it is difficult for the number of fundus images to be split at the ratio of 8:2 in the train dataset, the processor 220 may split fundus images at a ratio of 1:1. For example, when the number of fundus images in a specific stage (e.g., stage 6) is 2, the fundus images may be split into one training dataset of the train dataset and one test dataset of the train dataset.

In the case of normal patients, fundus images may be split such that a ratio of 1:1 is obtained to be the same as the age group of patients with moyamoya diseases.

FIG. 9 is a diagram for describing an embodiment of setting a fold dataset for a fundus image.

Referring to FIGS. 2 and 9, fundus images labeled as normal patients may be split into a fold dataset at a ratio of 1:n ('n' is an integer greater than or equal to 1) between the fundus images labeled as normal patients and the number of fundus images labeled as patients with moyamoya diseases, which are split for each age group. For example, the ratio of 1:n may be a ratio of 1:1, 1:2, or 1:3. However, an embodiment is not limited thereto.

Among the fundus images, the remaining fundus images used to create the test dataset of the train dataset may be used as the training dataset of the train dataset. All the fundus images labeled as moyamoya patients in the training dataset may be used for learning. Because the number of fundus images labeled as normal patients is greater than the number of fundus images labeled as moyamoya patients, 10 fold datasets may be randomly generated at a time such that the age group is multiplied by 'n'. The test dataset and fold dataset shown in FIG. 9 may be created.

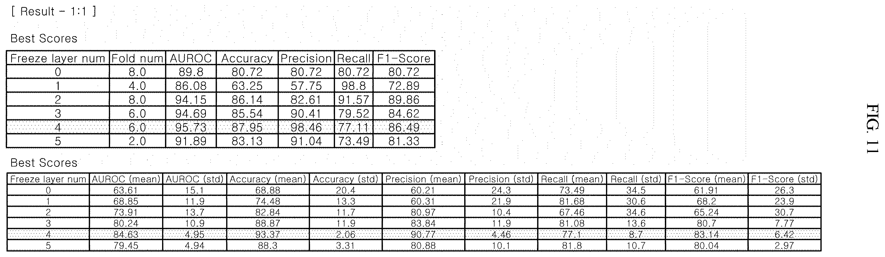

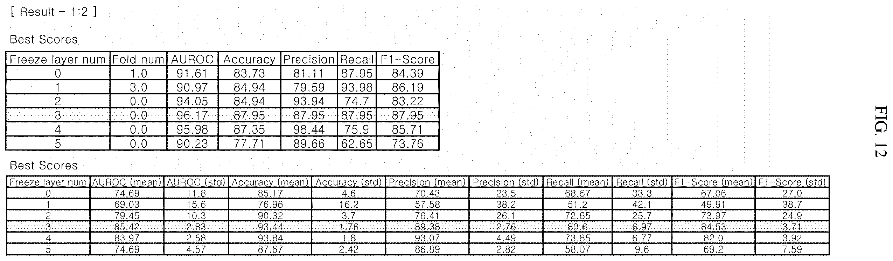

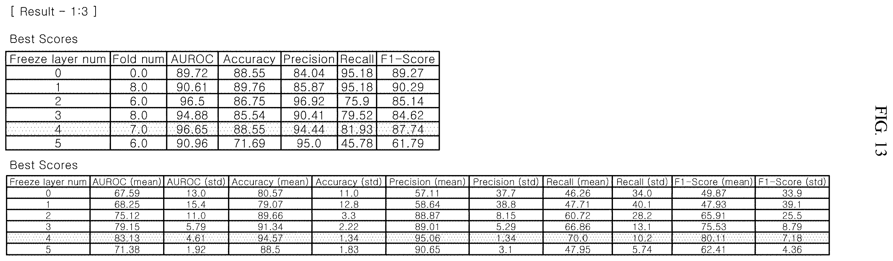

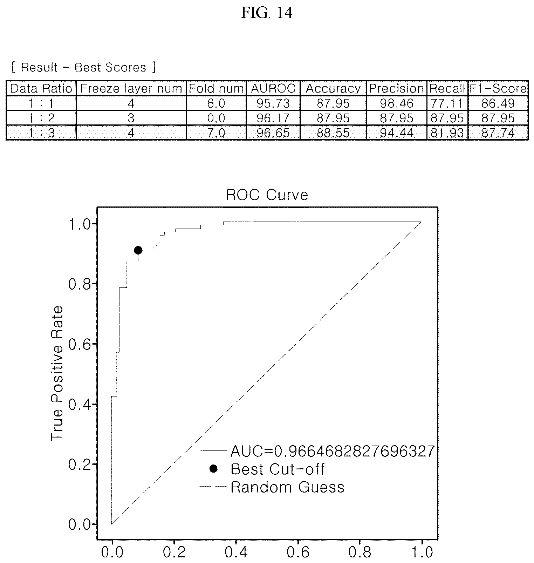

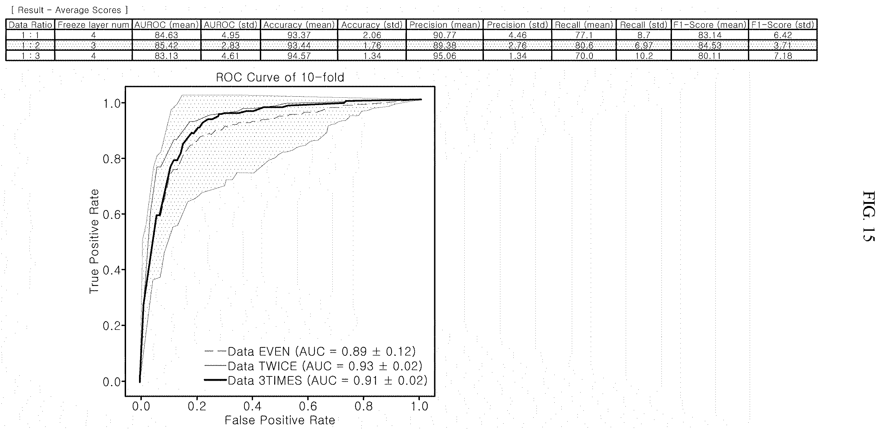

FIG. 10 is a diagram for describing a process of modeling an incidence prediction model. FIG. 11 is a diagram illustrating a score according to a data type indicating a ratio of 1:1. FIG. 12 is a diagram illustrating a score according to a data type indicating a ratio of 1:2. FIG. 13 is a diagram illustrating a score according to a data type indicating a ratio of 1:3. FIG. 14 is a diagram illustrating a ROC curve for each data type. FIG. 15 is a diagram illustrating a ROC curve for each data type according to a plurality of fold datasets.

Referring to FIG. 10, a data type may be a ratio of a fold dataset to fundus images labeled as normal patients. For example, the data type may include a data ratio of 1:1, a data ratio of 1:2, and a data ratio of 1:3.

Trasnform may include resizing a fundus image, adjusting a verticalflip of the fundus image, adjusting a color jitter of the fundus image, and adjusting a channel of lambda of the fundus image. In the meantime, a horizontalflip of the fundus image may not reflect a difference between the left and right eyes.

When an incidence prediction model is finetuned, the performance of the incidence prediction model may vary based on the range of a frozen layer. For example, the number of freezing layers for freezing layers may be 5.

A verification operation of a model may be performed. In this case, 20% of patients randomly split may follow the age distribution of the training dataset.

In some embodiments, the processor 220 may learn a plurality of incidence prediction models as many as 'n' data types, each of which indicates the data ratio of 1:n, the number of freezing layers, and the number of the fold dataset. Besides, the processor 220 may select an incidence prediction model, which has the highest AUROC, from among the plural incidence prediction models. For example, the incidence prediction models may be learned as many as the number (e.g., 150) corresponding to the product of 3 data types, 5 freezing layers, and 10 fold datasets. In this case, the incidence prediction model with the maximum AUROC may be selected from among 150 incidence prediction models.

In the data type of a data ratio of 1:1, a score for each freezing layer of the incidence prediction model learned depending on fold datasets may be calculated as shown in FIG. 11. In this case, best scores may indicate the highest AUROC calculated for each freezing layer. Average scores may indicate that average AUROC is calculated for each freezing layer.

In the data type of a data ratio of 1:2, best scores and average scores of the incidence prediction model learned for each freezing layer may be calculated as shown in FIG. 12.

In the data type of a data ratio of 1:3, best scores and average scores of the incidence prediction model learned for each freezing layer may be calculated as shown in FIG. 13.

The best score with the highest AUROC in each of the data ratio of 1:1, the data ratio of 1:2, and the data ratio of 1:3 shown in FIGS. 11 to 13 may be calculated as shown in FIG. 14.

The average score with the highest AUROC in each of the data ratio of 1:1, the data ratio of 1:2, and the data ratio of 1:3 shown in FIGS. 11 to 13 may be calculated as shown in FIG. 15.

FIG. 16 is a diagram showing fundus images for each stage. FIG. 17 is a diagram for describing a plurality of stages.

Referring to FIGS. 2 and 16, a fundus image may include nerves, ocular substances, and blood vessels. The Fundus image may vary for each age and for each stage. The fundus image may be preprocessed as shown in FIG. 16. The fundus image analyzed by Grad-CAM may be as shown in FIG. 16. The Grad-CAM may be a method used to interpret a model based on CNN. The image of blood vessels in the fundus image may be as shown in FIG. 16.

The processor 220 may determine the moyamoya disease based on at least one of the thickness of a blood vessel and the number of blood vessels included in the new fundus image of the moyamoya disease patient, and a predetermined reference value. In some embodiments, in normal patients, the processor 220 may determine a moyamoya disease based on the entire fundus image.

Referring to FIG. 17, a change in cerebral blood vessels may vary depending on stages. This change may be expected to be visible through a fundus. Accordingly, the binary classification shown in FIG. 17 may be added. The plurality of stages may include a stage 0 indicating normality, a stage 1 indicating stenosis of suprasellar internal carotid artery, a stage 2 indicating that stenosis of an internal carotid artery increases, and a moyamoya blood vessel is prominent, a stage 3 indicating that the moyamoya blood vessel in a brain is developed, a stage 4 indicating that occlusion of a circle of Willis and posterior cerebral artery begin, an extracranial collateral begins to appear, and the moyamoya blood vessel begins to disappear, a stage 5 indicating that a phenomenon according to the stage 4 further progresses, and a stage 6 indicating that the moyamoya blood vessel of a main cerebral artery completely disappears.

When the plurality of stages include stages 0 to 6, patients with moyamoya diseases according to stages 1 to 6 may be compared with normal patients. Alternatively, patients with moyamoya diseases according to stages 2 to 6 may be compared with the normal patients. Alternatively, patients with moyamoya diseases according to stages 3 to 6 may be compared with the normal patients. Alternatively, patients with moyamoya diseases according to stages 4 to 6 may be compared with the normal patients. Alternatively, patients with moyamoya diseases according to stages 5 to 6 may be compared with the normal patients.

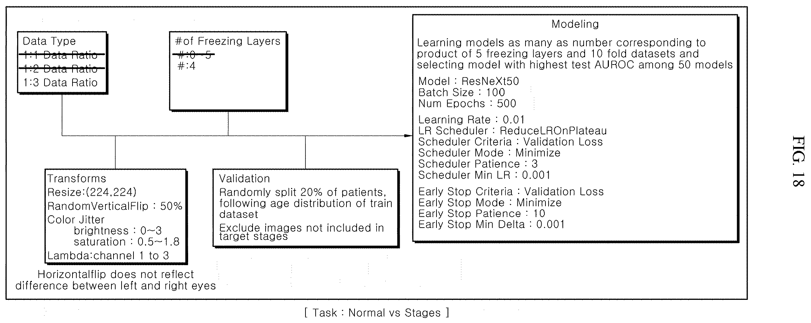

FIG. 18 is a diagram for describing a process of modeling an incidence prediction model according to a data type indicating a ratio of 1:3.

Referring to FIG. 18, similarly to those described above with reference to FIG. 10, after the data type is set, trasnform is performed, the number of freezing layers is determined, and validation is performed, an incidence prediction model may be modeled.

Unlike shown in FIG. 10, in FIG. 18, a data ratio of 1:3 may be used, and the incidence prediction model may be modeled based on a freezing layer 4. Accordingly, the incidence prediction model having the highest test AUROC may be selected from among the number of incidence prediction models corresponding to the product of 5 tasks and 10 fold datasets.

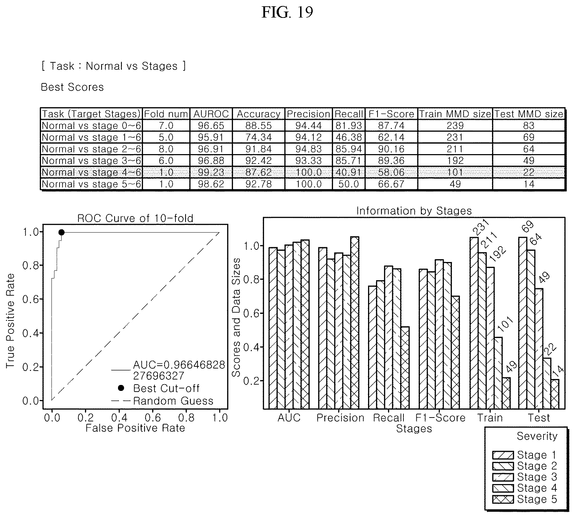

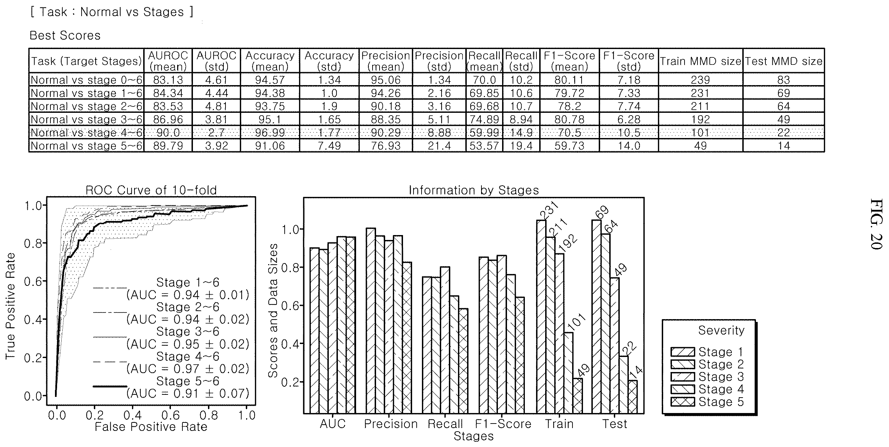

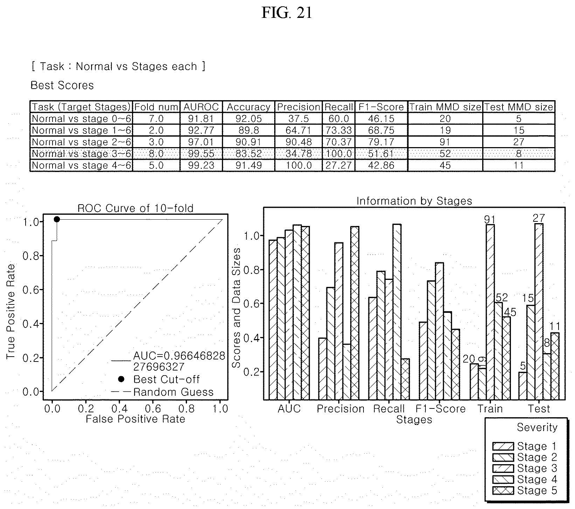

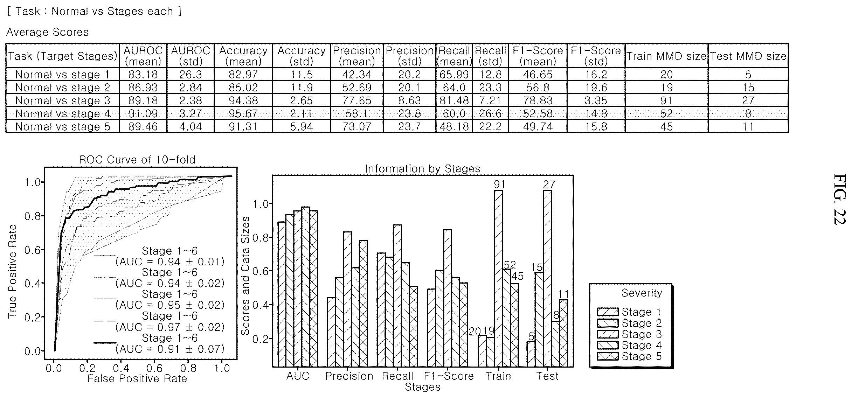

FIG. 19 is a diagram showing a best score for each target stage range. FIG. 20 is a diagram showing an average score for each target stage range. FIG. 21 is a diagram showing a best score for each target stage. FIG. 22 is a diagram showing an average score for each target stage.

Referring to FIGS. 18 and 19, a best score for normality and each target stage range (e.g., stages 0 to 6, stages 1 to 6, stages 2 to 6, stages 3 to 6, stages 4 to 6, or stages 5 to 6) may be improved. In this case, a task for normality and stages 4 to 6 may have the highest AUROC.

Referring to FIGS. 18 and 20, an average score for normality and each target stage range (e.g., stages 0 to 6, stages 1 to 6, stages 2 to 6, stages 3 to 6, stages 4 to 6, or stages 5 to 6) may be improved. In this case, a task for normality and stages 4 to 6 may have the highest AUROC.

Referring to FIGS. 18 and 21, the best score for normality and a target stage (e.g., any one of stages 1 to 6) may be improved. In this case, a task for normality and stage 4 may have the highest AUROC.

Referring to FIGS. 18 and 22, the average score for normality and a target stage (e.g., any one of stages 1 to 6) may be improved. In this case, a task for normality and stage 4 may have the highest AUROC.

Meanwhile, the disclosed embodiments may be implemented in a form of a recording medium storing instructions executable by a computer. The instructions may be stored in a form of program codes, and, when executed by a processor, generate a program module to perform operations of the disclosed embodiments. The recording medium may be implemented as a computer-readable recording medium.

The computer-readable recording medium may include all kinds of recording media in which instructions capable of being decoded by a computer are stored. For example, there may be read only memory (ROM), random access memory (RAM), magnetic tape, magnetic disk, flash memory, optical data storage device, and the like.

Disclosed embodiments are described above with reference to the accompanying drawings. One ordinary skilled in the art to which the present disclosure belongs will understand that the present disclosure may be practiced in forms other than the disclosed embodiments without altering the technical ideas or essential features of the present disclosure. The disclosed embodiments are examples and should not be construed as limited thereto.

According to the above-mentioned problem solving means of the present disclosure, the incidence of a moyamoya disease may be quickly and accurately predicted by using machine learning that learns retinal images.

Effects of the present disclosure are not limited to the effects mentioned above, and other effects not mentioned will be clearly understood by those skilled in the art from the following description.

## Claims

1. A server comprising:
a memory configured to store a deep learning-based incidence prediction model; and
a processor configured to predict an incidence probability of a moyamoya disease of a patient from a fundus image by using the incidence prediction model,
wherein the processor is configured to:
label input fundus images as a moyamoya disease patient with the moyamoya disease and a normal patient without the moyamoya disease;
learn the incidence prediction model by recognizing fundus images labeled as the moyamoya disease patient and fundus images labeled as the normal patient into the incidence prediction model; and
when a new fundus image is input, predict the incidence probability of the moyamoya disease by inputting the new fundus image into the learned incidence prediction model.

2. The server of claim 1, wherein the processor is configured to:
remove metadata and text data;
use only a fundus image including an optic disk for learning;
perform gray scaling on an image; and
preprocess the input fundus images by enhancing a contrast of an image.

3. The server of claim 2, wherein the processor is configured to:
split fundus images labeled in an order of stages, which have fewer patients for each age group, from among a plurality of stages classified depending on severity into a train dataset;
split 80% of fundus images labeled as the moyamoya disease patient into a training dataset of the train dataset, and split 20% of fundus images into a test dataset of the train dataset; and
split the fundus images labeled as the normal patient into the train dataset at a ratio of 1:1 between the number of the fundus images labeled as the normal patient and the number of the fundus images labeled as the moyamoya disease patient, which are split for each age group.

4. The server of claim 3, wherein the processor is configured to:
split the fundus images labeled as the normal patient into a fold dataset at a ratio of 1:n between the fundus images labeled as the normal patient and the number of the fundus images labeled as the moyamoya disease patient, which are split for each age group, wherein the 'n' is an integer greater than or equal to 1; and
generate the fold dataset including a plurality of fold datasets.

5. The server of claim 4, wherein the processor is configured to:
learn a plurality of incidence prediction models as many as 'n' data types, each of which indicates the ratio of 1:n, the number of freezing layers for freezing layers when the incidence prediction model is finetuned, and the number of the fold dataset; and
select an incidence prediction model having the highest area under receiver operating characteristic (AUROC) from among the plural incidence prediction models.

6. The server of claim 5, wherein the fundus image includes a nerve, an ocular substance, and a blood vessel, and
wherein the processor is configured to:
determine the moyamoya disease based on a predetermined reference value, and at least one of a thickness of the blood vessel included in the new fundus image of the moyamoya disease patient, and the number of the blood vessel.

7. The server of claim 6, wherein the processor is configured to:
determine the moyamoya disease based on the entire new fundus image of the normal patient.

8. The server of claim 7, wherein the plurality of stages include:
a stage 0 indicating normality;
a stage 1 indicating stenosis of suprasellar internal carotid artery;
a stage 2 indicating that stenosis of an internal carotid artery increases, and a moyamoya blood vessel is prominent;
a stage 3 indicating that the moyamoya blood vessel in a brain is developed;
a stage 4 indicating that occlusion of a circle of Willis and posterior cerebral artery begin, an extracranial collateral begins to appear, and the moyamoya blood vessel begins to disappear;
a stage 5 indicating that a phenomenon according to the stage 4 further progresses: and
a stage 6 indicating that the moyamoya blood vessel of a main cerebral artery completely disappears.

9. A computer-implemented method of predicting an incidence probability of a moyamoya disease in a patient from a fundus image by using a deep learning-based incidence prediction model, the method comprising:
labeling input fundus images as a moyamoya disease patient with the moyamoya disease and a normal patient without the moyamoya disease;
learning the incidence prediction model by recognizing fundus images labeled as the moyamoya disease patient and fundus images labeled as the normal patient into the incidence prediction model; and
predicting the incidence probability of the moyamoya disease by inputting a new fundus image thus input into the learned incidence prediction model.

10. The method of claim 9, further comprising:
removing metadata and text data;
using only a fundus image including an optic disk for learning;
performing gray scaling on an image; and
preprocessing the input fundus images by enhancing a contrast of an image.

11. The method of claim 10, wherein the learning of the incidence prediction model includes:
splitting fundus images labeled in an order of stages, which have fewer patients for each age group, from among a plurality of stages classified depending on severity into a train dataset.

12. The method of claim 11, wherein the learning of the incidence prediction model includes:
splitting 80% of fundus images labeled as the moyamoya disease patient into a training dataset of the train dataset, and split 20% of fundus images into a test dataset of the train dataset; and
splitting the fundus images labeled as the normal patient into the train dataset at a ratio of 1:1 between the number of the fundus images labeled as the normal patient and the number of the fundus images labeled as the moyamoya disease patient, which are split for each age group.

13. The method of claim 12, wherein the learning of the incidence prediction model includes:
splitting the fundus images labeled as the normal patient into a fold dataset at a ratio of 1:n between the fundus images labeled as the normal patient and the number of the fundus images labeled as the moyamoya disease patient, which are split for each age group, wherein the 'n' is an integer greater than or equal to 1; and
generating the fold dataset including a plurality of fold datasets.

14. The method of claim 13, wherein the learning of the incidence prediction model includes:
learning a plurality of incidence prediction models as many as 'n' data types, each of which indicates the ratio of 1:n, the number of freezing layers for freezing layers when the incidence prediction model is finetuned, and the number of the fold dataset; and
selecting an incidence prediction model having the highest AUROC from among the plural incidence prediction models.

15. The method of claim 14, wherein the fundus image includes a nerve, an ocular substance, and a blood vessel, and
wherein the predicting of the incidence probability of the moyamoya disease includes:
determining the moyamoya disease based on blood vessels included in a new fundus image of the moyamoya disease patient.

## Patentansprüche

1. Server, der aufweist:
einen Speicher, der dazu ausgebildet ist, ein auf Deep Learning basierendes Inzidenzvorhersagemodell zu speichern; und
einen Prozessor, der dazu ausgebildet ist, eine Inzidenzwahrscheinlichkeit einer Moyamoya-Krankheit eines Patienten aus Fundusbildern unter Verwendung des Inzidenzvorhersagemodells vorherzusagen,
wobei der Prozessor für Folgendes ausgebildet ist:
Kennzeichnen von eingegangenen Fundusbildern als einen Moyamoya-Krankheit-Patienten mit der Moyamoya-Krankheit und einen normalen Patienten ohne die Moyamoya-Krankheit;
Lernen des Inzidenzvorhersagemodells durch Erkennen von Fundusbildern, die als Moyamoya-Krankheit-Patient gekennzeichnet sind, und Fundusbildern, die als normaler Patient gekennzeichnet sind, in das Inzidenzvorhersagemodell; und
wenn ein neues Fundusbild eingegeben wird, Vorhersagen der Inzidenzwahrscheinlichkeit der Moyamoya-Krankheit durch Eingeben des neuen Fundusbildes in das erlernte Inzidenzwahrscheinlichkeitsmodell.

2. Server nach Anspruch 1, wobei der Prozessor für Folgendes ausgebildet ist:
Entfernen von Metadaten und Textdaten;
nur Verwenden eines Fundusbildes zum Lernen, das einen Sehnervenkopf enthält;
Durchführen einer Grauskalierung an dem Bild; und
Vorverarbeiten der eingegebenen Fundusbilder durch Verstärken eines Kontrasts eines Bildes.

3. Server nach Anspruch 2, wobei der Prozessor für Folgendes ausgebildet ist:
Aufteilen von Fundusbildern, die in einer Reihenfolge von Stadien gekennzeichnet sind, die für jede Altersgruppe weniger Patienten haben, aus einer Vielzahl von Stadien, die je nach Schweregrad klassifiziert sind, in einen Train-Datensatz;
Aufteilen von 80 % der Fundusbilder, die als Moyamoya-Krankheit-Patient gekennzeichnet sind, in einen Trainingsdatensatz des Train-Datensatzes, und Aufteilen von 20 % der Fundusbilder in einen Testdatensatz des Train-Datensatzes, und
Aufteilen der Fundusbilder, die als normaler Patient gekennzeichnet sind, in den Train-Datensatz in einem Verhältnis von 1: 1 zwischen der Anzahl von Fundusbildern, die als normaler Patient gekennzeichnet sind, und der Anzahl von Fundusbildern, die als Moyamoya-Krankheit-Patient gekennzeichnet sind, die für jede Altersgruppe aufgeteilt sind.

4. Server nach Anspruch 3, wobei der Prozessor für Folgendes ausgebildet ist:
Aufteilen der Fundusbilder, die als normaler Patient gekennzeichnet sind, in einen Fold-Datensatz in einem Verhältnis von 1:n zwischen den Fundusbildern, die als normaler Patient gekennzeichnet sind, und der Anzahl von Fundusbildern, die als Moyamoya-Krankheit-Patient gekennzeichnet sind, die für jede Altersgruppe aufgeteilt sind, wobei "n" eine ganze Zahl größer als oder gleich 1 ist; und
Erzeugen des Fold-Datensatzes, der eine Vielzahl von Fold-Datensätzen enthält.

5. Sever nach Anspruch 4, wobei der Prozessor für Folgendes ausgebildet ist:
Lernen einer Vielzahl von Inzidenzvorhersagemodellen so viele wie "n" Datentypen, wobei jedes das Verhältnis von 1:n, die Anzahl der Freezing-Schichten für Freezing-Schichten bei der Feinabstimmung des Inzidenzvorhersagemodells, und die Anzahl des Fold-Datensatzes angibt; und
Auswählen eines Inzidenzvorhersagemodells, das den höchsten Bereich unter der Receiver Operating Characteristic (AUROC) aufweist, aus der Vielzahl von Inzidenzvorhersagemodellen.

6. Server nach Anspruch 5, wobei das Fundusbild einen Nerv, eine okulare Substanz, und ein Blutgefäß umfasst, und
wobei der Prozessor für Folgendes ausgebildet ist:
Bestimmen der Moyamoya-Krankheit basierend auf einem vorbestimmten Referenzwert und mindestens einem aus einer Dicke des Blutgefäßes, das in dem neuen Fundusbild des Moyamoya-Krankheit-Patienten enthalten ist, und der Anzahl des Blutgefäßes.

7. Server nach Anspruch 6, wobei der Prozessor für Folgendes ausgebildet ist:
Bestimmen der Moyamoya-Krankheit basierend auf dem gesamten neuen Fundusbild des normalen Patienten.

8. Server nach Anspruch 7, wobei die Vielzahl von Stadien umfassen:
ein Stadium 0, das Normalität anzeigt;
ein Stadium 1, das eine Verengung der suprasellären inneren Halsschlagader anzeigt;
ein Stadium 2, das anzeigt, das eine Verengung einer inneren Halsschlagader zunimmt und ein Moyamoya-Blutgefäß prominent ist;
ein Stadium 3, das anzeigt, dass das Moyamoya-Blutgefäß in einem Gehirn entwickelt ist;
ein Stadium 4, das anzeigt, dass eine Okklusion eines Circle of Willis und der hintere Hirnschlagader beginnt, eine extrakranielle Kollaterale zu erscheinen beginnt, und das Moyamoya-Blutgefäß zu verschwinden beginnt;
ein Stadium 5, das anzeigt, dass ein Phänomen gemäß Stadium 4 weiter fortschreitet; und
ein Stadium 6, das anzeigt, dass ein Moyamoya-Blutgefäß einer Haupthirnschlagader vollständig verschwindet.

9. Computerimplementiertes Verfahren zur Vorhersage einer Inzidenzwahrscheinlichkeit einer Moyamoya-Krankheit bei einem Patienten aus einem Fundusbild unter Verwendung eines auf Deep Learning basierenden Inzidenzvorhersagemodells, wobei das Verfahren umfasst:
Kennzeichnen der eingegebenen Fundusbilder als einen Moyamoya-Krankheit-Patienten mit der Moyamoya-Krankheit und einen normalen Patienten ohne die Moyamoya-Krankheit;
Lernen des Inzidenzvorhersagemodells durch Erkennen von Fundusbildern, die als Moyamoya-Krankheit-Patient gekennzeichnet sind, und von Fundusbildern, die als normaler Patient gekennzeichnet sind, in das Inzidenzvorhersagemodell; und
Vorhersagen der Inzidenzwahrscheinlichkeit der Moyamoya-Krankheit durch Eingeben eines neuen Fundusbildes, das somit in das erlernte Inzidenzwahrscheinlichkeitsmodell eingegeben wird.

10. Verfahren nach Anspruch 9, das ferner umfasst:
Entfernen von Metadaten und Textdaten;
nur Verwenden eines Fundusbildes zum Lernen, das einen Sehnervenkopf enthält;
Durchführen einer Grauskalierung an dem Bild; und
Vorverarbeiten der eingegeben Fundusbilder durch Verstärken eines Kontrasts eines Bildes.

11. Verfahren nach Anspruch 10, wobei das Lernen des Inzidenzvorhersagemodells umfasst:
Aufteilen von Fundusbildern, die in einer Reihenfolge von Stadien gekennzeichnet sind, die für jede Altersgruppe weniger Patienten haben, aus einer Vielzahl von Stadien, die je nach Schweregrad klassifiziert sind, in einen Train-Datensatz.

12. Verfahren nach Anspruch 11, wobei das Lernen des Inzidenzvorhersagemodells umfasst:
Aufteilen von 80 % der Fundusbilder, die als Moyamoya-Krankheit-Patient gekennzeichnet sind, in einen Trainingsdatensatz des Train-Datensatzes, und Aufteilen von 20 % der Fundusbilder in einen Testdatensatz des Train-Datensatzes, und
Aufteilen der Fundusbilder, die als normaler Patient gekennzeichnet sind, in den Train-Datensatz in einem Verhältnis von 1: 1 zwischen der Anzahl von Fundusbildern, die als normaler Patient gekennzeichnet sind, und der Anzahl von Fundusbildern, die als Moyamoya-Krankheit-Patient gekennzeichnet sind, die für jede Altersgruppe aufgeteilt sind.

13. Verfahren nach Anspruch 12, wobei das Lernen des Inzidenzvorhersagemodells umfasst:
Aufteilen der Fundusbilder, die als normaler Patient gekennzeichnet sind, in einen Fold-Datensatz in einem Verhältnis von 1:n zwischen den Fundusbildern, die als normaler Patient gekennzeichnet sind, und der Anzahl von Fundusbildern, die als Moyamoya-Krankheit-Patient gekennzeichnet sind, die für jede Altersgruppe aufgeteilt sind, wobei "n" eine ganze Zahl größer als oder gleich 1 ist; und
Erzeugen des Fold-Datensatzes, der eine Vielzahl von Fold-Datensätzen enthält.

14. Verfahren nach Anspruch 13, wobei das Lernen des Inzidenzvorhersagemodells umfasst:
Lernen einer Vielzahl von Inzidenzvorhersagemodellen so viele wie "n" Datentypen, wobei jedes das Verhältnis von 1:n, die Anzahl der Freezing-Schichten für Freezing-Schichten bei der Feinabstimmung des Inzidenzvorhersagemodells, und die Anzahl des Fold-Datensatzes angibt; und
Auswählen eines Inzidenzvorhersagemodells mit der höchsten AUROC aus der Vielzahl von Inzidenzvorhersagemodellen.

15. Verfahren nach Anspruch 14, wobei das Fundusbild einen Nerv, eine okulare Substanz, und ein Blutgefäß umfasst, und
wobei das Vorhersagen der Inzidenzwahrscheinlichkeit der Moyamoya-Krankheit umfasst:
Bestimmen der Moyamoya-Krankheit basierend auf einem Blutgefäß, das in einem neuen Fundusbild des Moyamoya-Krankheit-Patienten enthalten ist.

## Revendications

1. Serveur, comprenant :
une mémoire configurée pour mémoriser un modèle de prédiction d'incidence basé sur un apprentissage profond ; et
un processeur configuré pour prédire une probabilité d'incidence d'une maladie de Moya-Moya chez un patient à partir d'une image de fond d'œil au moyen du modèle de prédiction d'incidence,
dans lequel le processeur est configuré pour :
étiqueter des images de fond d'œil entrées en tant que patient atteint de la maladie de Moya-Moya ayant la maladie de Moya-Moya et que patient normal n'ayant pas la maladie de Moya-Moya ;
apprendre le modèle de prédiction d'incidence par une reconnaissance d'images de fond d'œil étiquetées en tant que le patient atteint de la maladie de Moya-Moya et d'images de fond d'œil étiquetées en tant que le patient normal dans le modèle de prédiction d'incidence ; et
lorsqu'une nouvelle image de fond d'œil est entrée, prédire la probabilité d'incidence de la maladie de Moya-Moya en entrant la nouvelle image de fond d'œil dans le modèle de prédiction d'incidence appris.

2. Serveur selon la revendication 1, dans lequel le processeur est configuré pour :
retirer des métadonnées et des données textuelles ;
n'utiliser qu'une image de fond d'œil comprenant un disque optique à des fins d'apprentissage ;
appliquer une mise à l'échelle de gris à une image ; et
prétraiter les images de fond d'œil entrées par une amélioration d'un contraste d'une image.

3. Serveur selon la revendication 2, dans lequel le processeur est configuré pour :
diviser des images de fond d'œil étiquetées selon un ordre de stades, qui comportent moins de patients pour chaque groupe d'âge, parmi une pluralité de stades classifiés en fonction d'une sévérité en un ensemble de données à but d'entraînement ;
diviser 80 % d'images de fond d'œil étiquetées en tant que le patient atteint de la maladie de Moya-Moya en un ensemble de données d'entraînement de l'ensemble de données à but d'entraînement, et diviser 20 % d'images de fond d'œil en un ensemble de données de test de l'ensemble de données à but d'entraînement ; et
diviser les images de fond d'œil étiquetées en tant que le patient normal en l'ensemble de données à but d'entraînement selon un rapport de 1:1 entre le nombre d'images de fond d'œil étiquetées en tant que le patient normal et le nombre d'images de fond d'œil étiquetées en tant que le patient atteint de la maladie de Moya-Moya, qui sont divisées pour chaque groupe d'âge.

4. Serveur selon la revendication 3, dans lequel le processeur est configuré pour :
diviser les images de fond d'œil étiquetées en tant que le patient normal en un ensemble de données de facteur multiplicateur à un rapport de 1:n entre les images de fond d'œil étiquetées en tant que le patient normal et le nombre d'images de fond d'œil étiquetées en tant que le patient atteint de la maladie de Moya-Moya, qui sont divisées pour chaque groupe d'âge, dans lequel « n » est un nombre entier supérieur ou égal à 1 ; et
générer l'ensemble de données de facteur multiplicateur comprenant une pluralité d'ensembles de données de facteur multiplicateur.

5. Serveur selon la revendication 4, dans lequel le processeur est configuré pour :
apprendre une pluralité de modèles de prédiction d'incidence en tant qu'autant de « n » types de données, dont chacun indique le rapport de 1:n, le nombre de couches de gel de couches de gel lorsque le modèle de prédiction d'incidence est finement mis au point, et le nombre de l'ensemble de données de facteur multiplicateur ; et
sélectionner un modèle de prédiction d'incidence ayant la plus grande aire sous la courbe caractéristique de fonctionnement du récepteur (AUROC) parmi les plusieurs modèles de prédiction d'incidence.

6. Serveur selon la revendication 5, dans lequel l'image de fond d'œil comprend un nerf, une substance oculaire et un vaisseau sanguin, et
dans lequel le processeur est configuré pour :
déterminer la maladie de Moya-Moya sur la base d'une valeur de référence prédéterminée, et d'une épaisseur du vaisseau sanguin compris dans la nouvelle image de fond d"œil du patient atteint de la maladie de Moya-Moya et/ou du nombre de vaisseaux sanguins.

7. Serveur selon la revendication 6, dans lequel le processeur est configuré pour :
déterminer la maladie de Moya-Moya sur la base de toute la nouvelle image de fond d'œil du patient normal.

8. Serveur selon la revendication 7, dans lequel les stades de la pluralité de stades comprennent :
un stade 0 indiquant une normalité ;
un stade 1 indiquant une sténose d'une artère carotidienne interne supra-sellaire ;
un stade 2 indiquant qu'une sténose d'une artère carotidienne interne progresse, et qu'un vaisseau sanguin de Moya-Moya est proéminent ;
un stade 3 indiquant que le vaisseau sanguin de Moya-Moya est développé dans un cerveau ;
un stade 4 indiquant qu'une occlusion d'un cercle de Willis et d'une artère cérébrale postérieure commence, qu'une circulation collatérale extracrânienne commence à apparaître, et que le vaisseau sanguin de Moya-Moya commence à disparaître ;
un stade 5 indiquant qu'un phénomène selon le stade 4 progresse davantage ; et
un stade 6 indiquant que le vaisseau sanguin de Moya-Moya d'une artère cérébrale principale disparaît complètement.

9. Procédé mis en œuvre par ordinateur de prédiction d'une probabilité d'incidence d'une maladie de Moya-Moya chez un patient à partir d'une image de fond d'œil à l'aide d'un modèle de prédiction d'incidence basé sur un apprentissage profond, le procédé comprenant les étapes consistant à :
étiqueter des images de fond d'œil entrées en tant que patient atteint de la maladie de Moya-Moya ayant la maladie de Moya-Moya et que patient normal n'ayant pas la maladie de Moya-Moya ;
apprendre le modèle de prédiction d'incidence par une reconnaissance d'images de fond d'œil étiquetées en tant que le patient atteint de la maladie de Moya-Moya et d'images de fond d'œil étiquetées en tant que le patient normal dans le modèle de prédiction d'incidence ; et
prédire la probabilité d'incidence de la maladie de Moya-Moya en entrant une nouvelle image de fond d'œil ainsi entrée dans le modèle de prédiction d'incidence appris.

10. Procédé selon la revendication 9, comprenant en outre les étapes consistant à :
retirer des métadonnées et des données textuelles ;
n'utiliser qu'une image de fond d'œil comprenant un disque optique à des fins d'apprentissage ;
appliquer une mise à l'échelle de gris à une image ; et
prétraiter les images de fond d'œil entrées par une amélioration d'un contraste d'une image.

11. Procédé selon la revendication 10, dans lequel l'étape d'apprentissage du modèle de prédiction d'incidence consiste à :
diviser des images de fond d'œil étiquetées selon un ordre de stades, qui comportent moins de patients pour chaque groupe d'âge, parmi une pluralité de stades classifiés en fonction d'une sévérité en un ensemble de données à but d'entraînement.

12. Procédé selon la revendication 11, dans lequel l'étape d'apprentissage du modèle de prédiction d'incidence consiste à :
diviser 80 % d'image de fond d'œil étiquetées en tant que le patient atteint de la maladie de Moya-Moya en un ensemble de données d'entraînement de l'ensemble de données à but d'entraînement, et diviser 20 % d'images de fond d'œil en un ensemble de données de test de l'ensemble de données à but d'entraînement ; et
diviser les images de fond d'œil étiquetées en tant que le patient normal en l'ensemble de données à but d'entraînement selon un rapport de 1:1 entre le nombre d'images de fond d'œil étiquetées en tant que le patient normal et le nombre d'images de fond d'œil étiquetées en tant que le patient atteint de la maladie de Moya-Moya, qui sont divisées pour chaque groupe d'âge.

13. Procédé selon la revendication 12, dans lequel l'étape d'apprentissage du modèle de prédiction d'incidence consiste à :
diviser les images de fond d'œil étiquetées en tant que le patient normal en un ensemble de données de facteur multiplicateur à un rapport de 1:n entre les images de fond d'œil étiquetées en tant que le patient normal et le nombre d'images de fond d'œil étiquetées en tant que le patient atteint de la maladie de Moya-Moya, qui sont divisées pour chaque groupe d'âge, dans lequel « n » est un nombre entier supérieur ou égal à 1 ; et
générer l'ensemble de données de facteur multiplicateur comprenant une pluralité d'ensembles de données de facteur multiplicateur.

14. Procédé selon la revendication 13, dans lequel l'étape d'apprentissage du modèle de prédiction d'incidence consiste à :
apprendre une pluralité de modèles de prédiction d'incidence en tant qu'autant de « n » types de données, dont chacun indique le rapport de 1:n, le nombre de couches de gel de couches de gel lorsque le modèle de prédiction d'incidence est finement mis au point, et le nombre de l'ensemble de données de facteur multiplicateur ; et
sélectionner un modèle de prédiction d'incidence ayant la plus grande AUROC parmi les plusieurs modèles de prédiction d'incidence.

15. Procédé selon la revendication 14, dans lequel l'image de fond d'œil comprend un nerf, une substance oculaire et un vaisseau sanguin, et
dans lequel la prédiction de la probabilité d'incidence de la maladie de Moya-Moya consiste à :
déterminer la maladie de Moya-Moya sur la base de vaisseaux sanguins compris dans une nouvelle image de fons d'œil du patient atteint de la maladie de Moya-Moya.
